# EUROPÄISCHE PATENTSCHRIFT

(11) **EP 0 559 306 B1**
(45) Veröffentlichungstag und Bekanntmachung des Hinweises auf die Patenterteilung: **28.05.1997**
(21) Anmeldenummer: 93250067.1
(22) Anmeldetag: 02.03.1993
(51) Int. Cl.: G01N 33/00, G01D 18/00

(54) **Schaltungsanordnung zum Nullabgleich und zur Empfindlichkeitseinstellung eines analogen Signals bei Messeinrichtungen**
Circuit for zeroing and sensitivity adjustment of an analog signal in measuring equipment
Dispositif de calibrage à zéro et réglage de la sensibilité d'un signal analogue pour dispositifs de mesure

(30) Priorität: 03.03.1992 DE 4206637
(43) Veröffentlichungstag der Anmeldung: 08.09.1993
(73) Patentinhaber: AUERGESELLSCHAFT GMBH, D-12059 Berlin (DE)
(72) Erfinder: Lemke, Andreas, W-1000 Berlin 41 (DE)

(56) Entgegenhaltungen:
- DE-B- 2 437 438
- US-A- 4 348 732

## Beschreibung

Die Erfindung betrifft eine Schaltungsanordnung zum Nullabgleich und zur Empfindlichkeitseinstellung nach dem Oberbegriff des Anspruchs 1.

Mit einem Nullabgleich wird beispielsweise in Brücken- oder Kompensationsschaltungen ein Spannungsloser Zustand des Nullzweiges zur Erfassung von Werten der Meßgröße bewirkt. Hierfür wird zur Durchführung des Nullabgleichs ein auf einen Meßwertgeber aufzugebendes Nullgas (synthetische Luft oder Frischluft ohne Anteil der Meßkomponente) benutzt, wobei der Meßwert auf Null einlaufen muß. Bei Abweichungen des Meßwertes muß dieser auf Null eingestellt werden. In der Schaltungsanordnung muß dafür wenigstens ein einstellbares Bauelement vorhanden sein, mit dem sich dieses Ziel erreichen läßt. Für die Einstellung der Empfindlichkeit einer Meßeinrichtung wird ein auf den Meßwertgeber aufgegebenes Prüfgas bekannter Konzentration benutzt, wobei die entsprechende Prüfgaskonzentration in einer Anzeige erscheint. Bei Abweichungen muß ebenfalls mit Hilfe eines in der Schaltungsanordnung vorhandenen Einstellelementes auf den Prüfgaskonzentrations-Sollwert eingestellt weroen. Es ist bekannt, daß beispielsweise der Nullabgleich und die Empfindlichkeitseinstellung jeweils mittels eines von Hand einzustellenden Potentiometers erfolgen kann, was ungenau und personalintensiv ist.

Aus der DE-AS 24 37 438 ist eine Schaltungsanordnung zum Nullabgleich und zur Einstellung der Empfindlichkeit bei Meßeinrichtungen mit einem Meßwertgeber, dessen Signal in einem Meßsignalverstärker verstärkt wird und dem ein AD-Wandler und eine Signalaufbereitungsanlage sowie eine Anzeige nachgeschaltet ist, bekannt. Gemäß diesem Stand der Technik ist an die Signalaufbereitungsanlage eine Nullpunkteinstellstufe und eine Empfindlichkeitseinstellstufe geschaltet, wobei in der Signalaufbereitungsanlage jeweils ein Null- und ein Referenzwert als Sollwert einer festgelegten Ausgangsgröße für den Nullabgleich und den Empfindlichkeitsabgleich abgelegt sind. Die Lösung hat den Nachteil, daß ein Vergleich der Meßwerte immer nur mit dem einen festgelegten Referenzwert für den Nullabgleich und den Empfindlichkeitsabgleich möglich ist. Eine Nachregelung kann nicht erfolgen. Gleichermaßen erfolgt die Nullpunkteinstellung und Empfindlichkeitseinstellung gemäß US-PS 4 348 732. Da die Meßwerte auch bei dieser Lösung nur mit einmal festgelegten Referenzwerten für den Nullpunkt und für die Empfindlichkeit verglichen werden, kann eine Nachregelung auftretender Driften des Nullpunktes oder der Empfindlichkeit während des Meßprozesses nicht ausgeglichen werden.

Der Erfindung liegt daher die Aufgabe zugrunde, eine einfache, preisgünstige Schaltung für den Nullabgleich und für die Einstellung der Empfindlichkeit zu schaffen, die einerseits keine Potentiometer erfordert und andererseits einen automatischen Nullabgleich und eine automatische Einstellung der Empfindlichkeit ermöglicht.

Diese Aufgabe wird durch die kennzeichnenden Merkmale des Anspruchs 1 gelöst.

Vorteilhafte Weiterbildungen der Erfindung sind in abhängigen Ansprüchen gekennzeichnet.

Ein Ausführungsbeispiel der Erfindung ist in der Zeichnung dargestellt und wird im folgenden näher beschrieben.

Es zeigt
- Fig. 1: ein Blockschaltbild der erfindungsgemäßen Schaltungsanordnung zum Nullabgleich und zur Einstellung der Empfindlichkeit
- Fig. 2: ein Blockschaltbild der Stellglieder zur Einstellung des Nullpunktes und der Empfindlichkeit, und
- Fig. 3: einen Stromlaufplan nach Fig. 1

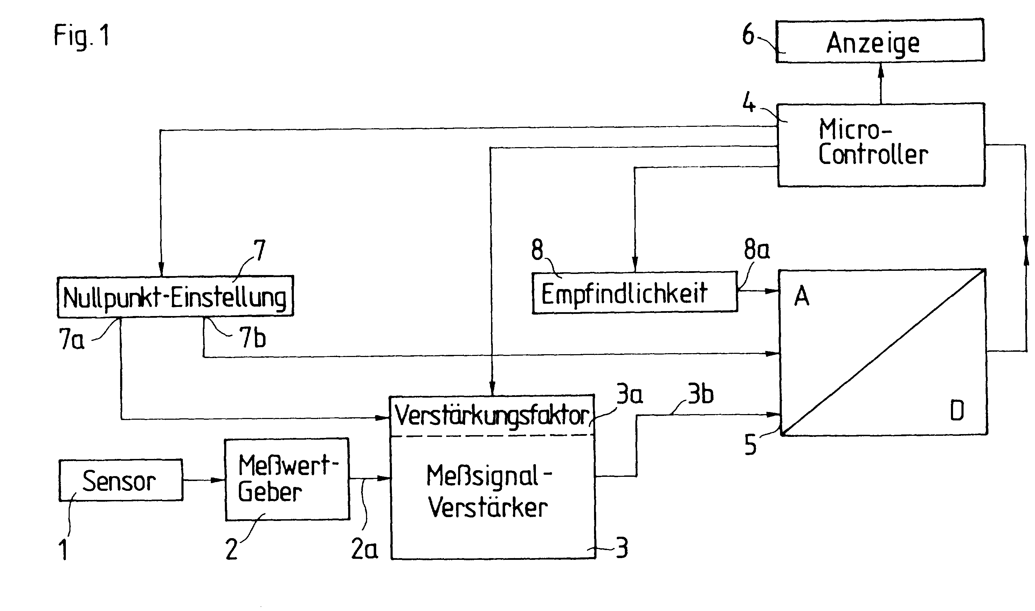

Wie aus Fig. 1 ersichtlich ist, besteht die als Blockschaltbild dargestellte Schaltungsanordnung aus einem Sensor 1, einem Meßwertgeber 2, der ein Sensorsignal 2 a erzeugt, das zur Weiterverarbeitung auf einen mit dem Meßwertgeber 2 in Reihe geschalteten Meßsignal-Verstärker 3 geschaltet ist, wobei dieser eine Verstärkungsstufe 3 a mit einem variablen Verstärkungsfaktor aufweist. Das vom Meßsignal-Verstärker 3 verstärkte Sensorsignal 3 b gelangt zur Auswertung auf eine Signalaufbereitungsanlage 4, der ein AD-Wandler 5 vorgeschaltet ist, da für die Verarbeitung der analogen Sensorsignale 3 b durch die Signalaufbereitungsanlage jedes Signal digitalisiert werden muß. Die Signalaufbereitungsanlage 4 ist in vorteilhafter Weise ein Microcontroller. Die durch geeignete Verknüpfung im Microcontroller 4 verarbeiteten Sensorsignale 3 b gelangen als Meßwerte auf eine dem Micocontroller nachgeschaltete LCD-Anzeige 6.

Zur Einstellung des erforderlichen Meßsignal-Nullpunktes für die Meßeinrichtung, die vor jeden Meßvorgang erfolgt, und für die nachfolgende Einstellung der Empfindlichkeit, dient jeweils eine Nullpunkt-Einstellung 7 und eine Empfindlichkeits-Einstellung 8. Beide Einstellungsteile 7 und 8 sind eingangsseitig mit dem Microcontroller 4 verbunden und werden von diesem gesteuert. Ausgangsseitig sind die Einstellungsteile 7 und 8 mit dem AD-Wandler 5 schaltungstechnisch über die Ausgänge 7b und 8a verbunden, wobei die Nullpunkt-Einstellung 7 noch einen weiteren Ausgang 7a aufweist, der mit dem MeßsignalVerstärker 3 verbunden ist. Der Meßsignalverstärker 3 weist einen vorgegebenen Aussteuerbereich auf, innerhalb dessen die Meßeinrichtung anzeigt. Mit der vom Microcontroller 4 gesteuerten Nullpunkt-Einstellung 7 kann der Meßsignal-Nullpunkt innerhalb des gesamten Aussteuerbereiches des Meßsignal-Verstärkers 3 eingestellt werden. Dadurch können Sensorsignale verarbeitet werden, die mit zunehmender Konzentration der in Luft enthaltenen Gase und Dämpfe ansteigende oder, mit abnehmender Konzentration, abfallende Meßsignale abgeben.
Der Meßsignalverstärker 3 wird ebenfalls über die Verstärkungsstufe 3 a durch den Microcontroller 4 gesteuert und von diesem so eingestellt, daß bei einem maximalen Sensorsignal 3 b der Aussteuerbereich des Meßsignal-Verstärkers 3 nicht überschritten werden kann. Dies wird durch den variablen Verstärkungsfaktor der Verstärkerstufe 3a erreicht.

Die Empfindlichkeits-Einstellung 8 wird durch den Microcontroller 4 gesteuert und so eingestellt, daß an der Anzeige 6 als Anzeigeendwert der Meßbereich plus ein einstellbarer Überlauf angezeigt werden kann. Durch diese Maßnahme wird die Anzeigegenauigkeit innerhalb des Meßbereiches erhöht. Der Meßbereich der digitalen Anzeige 6 erfährt eine Erweiterung und kann sicher überschritten werden. Durch die vom Microcontroller 4 gesteuerte Empfindlichkeits-Einstellung 8 kann für verschiedene Meßbereiche stets eine maximale Auflösung des Meßsignals erreicht werden, weil die jeweilige Empfindlichkeits-Meßspannung variabel einstellbar ist.
Zur Einstellung der Empfindlichkeit 8: Im Microcontroller 4 werden als Kalibrierdaten für die Einstellung der Empfindlichkeit Prüfgaskonzentrationswerte C als Sollwerte einer Ausgangsgröße abgelegt, die den Meßbereichsendwert der Prüfgaskonzentration darstellen und bei Abweichungen an den Sollwert angepaßt werden. Zum Einstellen der Empfindlichkeit wird der Meßwertgeber 2 mit einem Prüfgas bekannter Konzentration C beaufschlagt. Nach Betätigen einer in der Zeichnung nicht dargestellten Taste wird das der Prüfgaskonzentration entsprechende Signal 3 b, welches über den AD-Wandler 5 an den Microcontroller 4 geliefert wird, mit dem abgelegten Sollwert verglichen und bei Abweichungen ein neuer Faktor berechnet, der der veränderten Empfindlichkeit entspricht.

Zur Einstellung des Nullpunktes 7: Im Microcontroller 4 wird der festgelegte Nullgaskonzentrationswert Co als Sollwert abgelegt, der auf der Anzeige 6 den Anzeigewert "NULL" ergibt. Nach Beaufschlagung des Meßwertgebers 2 mit einem Nullgas, wird ein der Nullkonzentration Co entsprechendes Signal So (3 b) an den Microcontroller 4 geliefert. Dort erfolgt ein Vergleichen mit dem abgelegten Sollwert der Nullkonzentration Co. Bei Abweichungen wird mit Hilfe der Nullpunkt-Einstellung 7 das Signal So so verschoben (eingestellt), daß es dem internen festgelegten Nullkonzentrationswert Co entspricht. Der so ermittelte Wert für die Nullpunkt-Einstellung 7 wird im Microcontroller 4 abgelegt.

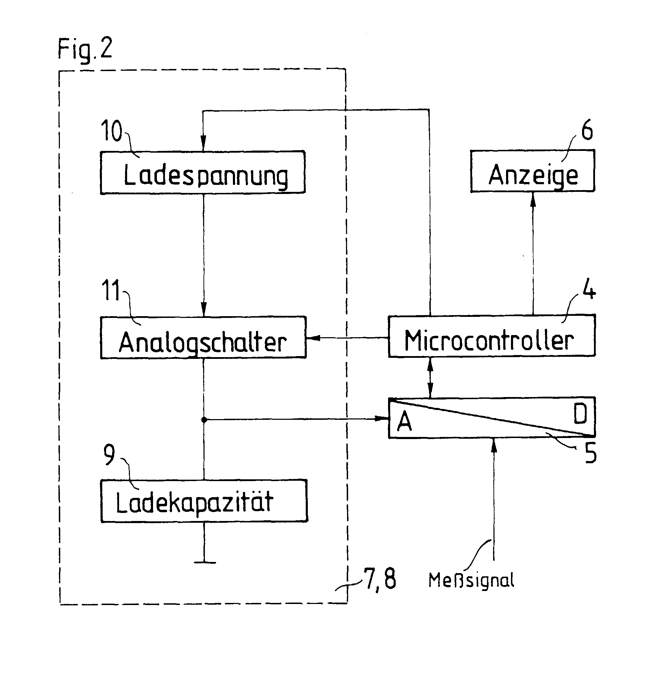

In Fig. 2 ist der Schaltungsaufbau für die Nullpunkt- und Empfindlichkeits-Einstellung 7 und 8 als Blockschaltbild dargestellt. Beide Einstellungen sind in ihrem Aufbau identisch und verwenden gleiche Stellglieder. Die Stellglieder zur Anpassung des Nullpunktes oder der Empfindlichkeit an den AD-Wandler 5 bestehen jeweils aus einer Ladekapazität 9, einem Widerstand 10, der eine im Spannungspegel wählbare Ladespannung aufzunehmen vermag, und aus einem dem Widerstand 10 vorgeschalteten Analogschalter 11. Die Ladekapazität 9 wird zum Ent- und Aufladen durch den Microcontroller 4 gesteuert. Der Microcontroller 4 mißt über den AD-Wandler 5 den Spannungswert, auf den die Ladekapazität 9 aufgeladen ist. Wenn der gemessene Spannungswert nicht dem im Microcontroller 4 abgelegten Sollwert für die Nullpunktspannung oder die Empfindlichkeitsspannung entspricht, dann steuert der Microcontroller 4 über die Ladespannung 10 und den Analogschalter 11 solange Lade- und/oder Entladevorgänge der Ladekapazität 9, bis der entsprechende Sollwert für den Nullpunkt oder für die Empfindlichkeit erreicht wird.
Die Ermittlung der entsprechenden Sollwerte für den Nullpunkt und für die Empfindlichkeit erfolgt durch Kalibrierung mit einem Nullgas ohne Anteil der Meßkomponenten und mit einem Prüfgas bekannter Konzentration während eines vom Microcontroller 4 gesteuerten Zeitablaufes, und zwar in der Reihenfolge Nullpunkt-Einstellung und dann Empfindlichkeits-Einstellung. Der Sollwert für den Signalnullpunkt ist innerhalb des gesamten festgelegten Aussteuerbereichs des Meßsignalverstärkers 3 aufgrund seines variablen Verstärkungsfaktors verschiebbar.

Dieser Aussteuerbereich kann beispielsweise einen Bereich von 0 V bis 5 V Signalspannung umfassen. Der Sollwert der Empfindlichkeits-Meßspannung ist ebenfalls zur Anpassung des analogen Meßsignals an den AD-Wandler 5 innerhalb eines variablen Meßbereiches, durch die Empfindlichkeits-Einstellung 8, einstellbar. Auf diese Weise ist vorteilhaft immer eine maximale Auflösung des Analogsignals gegeben.

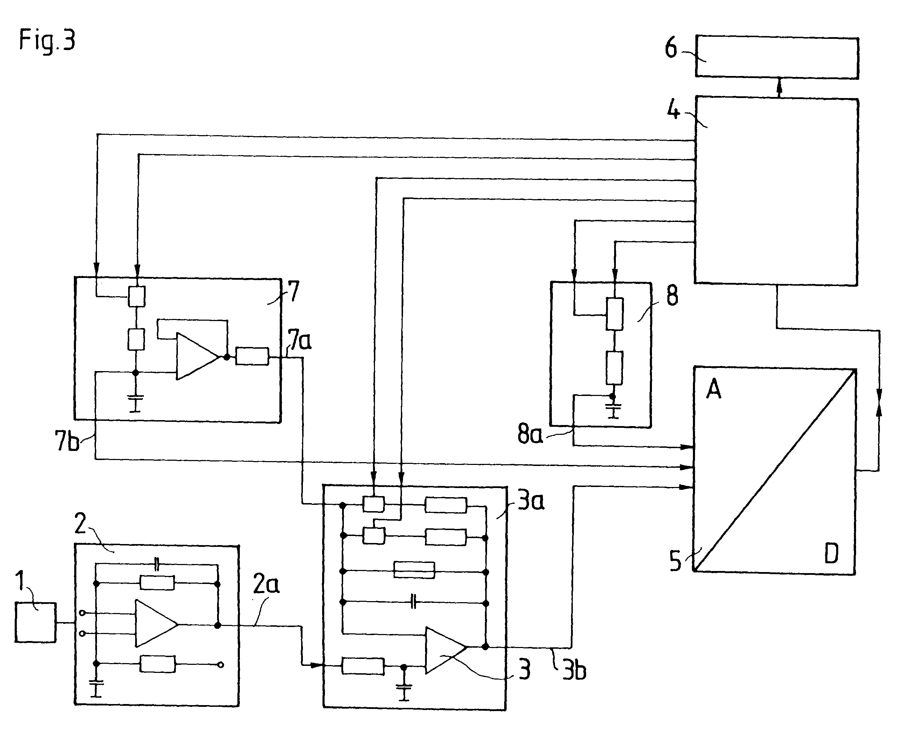

In Fig. 3 ist der Stromlaufplan dargestellt, in dem die Schaltungsanordnung mit ihrem Verbindungsaufbau für den automatischen Nullabgleich und für die Einstellung der Empfindlichkeit eines analogen Meßsignals nach der erfindungsgemäßen Meßeinrichtung zu entnehmen ist.

## Patentansprüche

1. Schaltungsanordnung zum Nullabgleich und zur Einstellung der Empfindlichkeit bei Meßeinrichtungen mit einem Meßwertgeber (2), dessen Meßsignal in einem Meßsignal-Verstärker (3) verstärkt wird, und dem ein AD-Wandler (5) und eine Signalaufbereitungsanlage (4) sowie eine digitale Anzeige (6) nachgeschaltet ist, wobei an die Signalaufbereitungsanlage (4) eine Nullpunkt-Einstellung (7) und eine Empfindlichkeits - Einstellung (8) geschaltet ist, dadurch gekennzeichnet, daß zum Nullabgleich und zur Einstellung der Empfindlichkeit von Brücken - oder Kompensationschaltungen,
a) die Nullpunkt-Einstellung (7) mit einem Ausgang (7 a) mit einer variablen Verstärkungsstufe (3 a) des Meßsignal-Verstärkers (3) verbunden ist, so daß dadurch der Signal-Nullpunkt innerhalb eines vorgegebenen Aussteuerbereiches des Verstärkers (3) einstellbar und/oder verschiebbar ist, und ein weiterer Ausgang (7 b) der Nullpunkt-Einstellung (7) für einen Anschluß an den AD-Wandler (5) vorgesehen ist,
b) die Empfindlichkeits-Einstellung (8) mit ihrem Ausgang (8 a) mit dem AD-Wandler (5) verbunden ist, und die von der Signalaufbereitungsanlage (4) gesteuert und so eingestellt wird, daß die zum Wandeln des analogen Meßsignals erforderliche Referenzspannung des AD-Wandlers (5) zur Anpassung an das Meßsignal innerhalb des vorgegebenen Meßbereichs der Meßeinrichtung variabel einstellbar ist, und
c) in der Signalaufbereitungsanlage (4) jeweils zur Konzentrationsmessung von gasen benötigten Nullgas- und Prüfgaskonzentrationswerte (Co und C) als Sollwerte einer festgelegten Ausgangsgröße für den Nullabgleich und die Empfindlichkeits-Einstellung abgelegt und dort mit den Istwerten der gemessenen Gaskonzentrationen vergleichbar und korrigierbar sind.

2. Schaltungsanordnung nach Anspruch 1, dadurch gekennzeichnet, daß jeder Stellglied (7, 8) zur Anpassung des Nullpunktes oder der Empfindlichkeit an den AD-Wandler (5) aus einer mit dem AD-Wandler verbundenen Ladekapazität (9) besteht, die über einen Widerstand (10) mit einem diesen vorgeschalteten Analogschalter (11) an die Signalverarbeitungsanlage (4) geschaltet ist.

3. Schaltungsanordnung nach Anspruch 2, dadurch gekennzeichnet, daß die Ladekapazität (9) zum Ent- und Aufladen durch die Signalverarbeitungsanlage (4) steuerbar ist, wobei diese über den Widerstand (10) und den Analogschalter (11) solange Lade- und/oder Entladevorgänge der Ladekapazität (9) steuert, bis der entsprechende Sollwert für den Nullpunkt oder für die Empfindlichkeit erreicht ist.

4. Schaltungsanordnung nach Anspruch 1, dadurch gekennzeichnet, daß die durch die Signalverarbeitungsanlage (4) gesteuerte Empfindlichkeitsspannung so eingestellt wird, daß an der Anzeige (6) als Anzeigeendwert der Meßbereich plus ein einstellbarer Überlauf anzeigbar ist.

5. Schaltungsanordnung nach Anspruch 1, dadurch gekennzeichnet, daß die Signalverarbeitungsanlage (4) ein Microcontroller ist.

6. Schaltungsanordnung nach Anspruch 1, zur Einstellung und Anpassung eines in Wert und Polarität variablen Analogsignals an den AD-Wandler, dadurch gekennzeichnet, daß
a) zur Anpassung des Analogsignals Stellglieder (7, 8) vorgesehen sind, die jeweils aus einer mit dem AD-Wandler (5) verbundenen Ladekapazität (9) besteht, die über einen Widerstand (10) mit einem diesen vorgeschalteten Analogschalter (11) an die Signalverarbeitungsanlage (4) geschaltet ist,
b) die Signalverarbeitungsanlage (4) über den AD-Wandler (5) den Spannungswert der Kapazität (9) meßbar macht, und
c) die Ladekapazität (9) zum Ent- und Aufladen durch die Signalverarbeitungsanlage (4) steuerbar ist, wobei diese über den Widerstand (10) und den Analogschalter (11) solange Lade- und/oder Entladevorgänge der Ladekapazität (9) steuert, bis der gemessene Spannungswert dem Sollwert des Analogsignals entspricht .

## Revendications

1. Configuration de circuit destinée à compenser et à régler la sensibilité de systèmes de mesure et comportant un capteur de mesure (2) dont le signal de mesure est amplifié dans un amplificateur de signaux de mesure (3), un transducteur analogique-numérique (5) et un dispositif de mise en forme de signaux (4) ainsi qu'un affichage numérique (6) étant intercalés à la suite de ce capteur tandis qu'un réglage de zéro (7) et un réglage de sensibilité (8) sont branchés au dispositif de mise en forme de signaux (4), ladite configuration étant caractérisée par le fait que pour compenser et régler la sensibilité de circuits en pont ou de circuits de compensation
a) le réglage de zéro (7) est branché par une sortie (7 a) à un plot d'amplification (3 a) de l'amplificateur de signaux de mesure (3), de sorte que le zéro du signal peut être réglé et/ou déplacé à l'intérieur d'une zone de modulation prédéfinie de l'amplificateur (3), et une autre sortie (7 b) du réglage de zéro (7) est prévue pour un raccordement au transducteur analogique-numérique (5),
b) le réglage de sensibilité (8) est branché par sa sortie (8 a) au transducteur analogique-numérique (5), et qu'il est commandé et réglé par un dispositif de mise en forme de signaux (4) de façon à ce que la tension de référence du transducteur analogique-numérique (5) nécessaire à la transduction du signal de mesure analogique puisse être réglée de manière variable pour l'adapter au signal de mesure à l'intérieur de la zone de mesure prédéfinie du système de mesure, et
c) dans le dispositif de mise en forme de signaux (4), les valeurs de concentration de gaz détendu à pression atmosphérique et de gaz d'essai (Co et C) nécessaires à la mesure de la concentration des gaz sont respectivement enregistrées en tant que valeurs de consigne d'une grandeur de sortie prédéterminée pour la compensation et le réglage de la sensibilité, et qu'elles puissent être comparées aux valeurs réelles des concentrations de gaz mesurées, et corrigées le cas échéant.

2. Configuration de circuit selon revendication 1, ladite configuration étant caractérisée par le fait que chacun des éléments de réglage (7, 8) pour l'adaptation du zéro ou de la sensibilité au transducteur analogique-numérique (5) est constitué d'une capacité de charge (9) reliée au transducteur analogique-numérique qui est branchée au dispositif de mise en forme de signaux (4) via une résistance (10) par un commutateur analogique (11) couplé en amont de ce dernier.

3. Configuration de circuit selon revendication 2, ladite configuration étant caractérisée par le fait que la capacité de charge (9) peut être commandée pour la décharge et la charge par le dispositif de mise en forme de signaux (4), ce dernier commandant les opérations de charge et/ou de décharge de la capacité de charge (9) via la résistance (10) et le commutateur analogique (11) jusqu'à ce que la valeur de consigne correspondante pour le zéro ou pour la sensibilité soit atteinte.

4. Configuration de circuit selon revendication 1, ladite configuration étant caractérisée par le fait que la tension de sensibilité commandée par le dispositif de mise en forme de signaux (4) est réglée de façon à ce que sur l'affichage (6), la zone de mesure plus un dépassement réglable puissent être affichés en tant que valeur finale d'affichage.

5. Configuration de circuit selon revendication 1, ladite configuration étant caractérisée par le fait que le dispositif de mise en forme de signaux (4) est un microcontrôleur.

6. Configuration de circuit selon revendication 1 pour le réglage et l'adaptation d'un signal analogique de valeur et de polarité variables au transducteur analogique-numérique, ladite configuration étant caractérisée par le fait que
a) pour l'adaptation du signal analogique, des éléments de réglage (7, 8) respectivement constitués d'une capacité de charge (9) reliée au transducteur analogique-numérique (5) qui est branchée au dispositif de mise en forme de signaux (4) via une résistance (10) par un commutateur analogique (11) couplé en amont de ce dernier sont prévus,
b) le dispositif de mise en forme de signaux (4) permet de mesurer la valeur de tension de la capacité (9) via le transducteur analogique-numérique (5), et
c) la capacité de charge (9) peut être commandée pour la décharge et la charge par le dispositif de mise en forme de signaux (4), ce dernier commandant les opérations de charge et/ou de décharge de la capacité de charge (9) via la résistance (10) et le commutateur analogique (11) jusqu'à ce que la valeur de tension mesurée corresponde à la valeur de consigne du signal analogique.

## Claims

1. Circuit configuration for zero balancing and for adjusting the sensitivity in measuring instruments with a data transmitter (2), the measuring signal of which is amplified in a measuring amplifier (3), connected in the outgoing circuit to an AD transducer (5) and a signal conditioning unit (4) as well as a digital display (6), with a zero adjust (7) and a sensitivity adjust (8) connected to the signal conditioning unit (4), characterized by the fact, that for zero balancing and for adjusting the sensitivity of bridge or compensation circuits,
a) the zero adjust (7) is connected via one output (7a) to a variable amplification stage (3a) of the measuring amplifier (3), so that the signal zero point can therefore be set and/or adjusted within a given range of control of the amplifier (3), and the zero adjust (7) is provided with an additional output (7b) for connection to the AD transducer (5),
b) the sensitivity adjust (8) is connected via its output (8a) to the AD transducer (5), and it is controlled and set in such a way by the signal conditioning unit (4) that the reference voltage of the AD transducer (5) needed to transduce the analogue measuring signal can be varied to match the measuring signal of the measuring equipment within the given measuring range, and
c) the respective concentration values for the zero gas and span gas (Co and C) needed to measure gas concentrations can be stored in the signal conditioning unit (4) as setpoint values of a specified output for the zero balance and the sensitivity adjustment, as well as compared and corrected there with the actual values of the measured gas concentrations.

2. Circuit configuration according to claim 1, characterized by the fact, that each of the control elements (7, 8) for matching the zero point or the sensitivity to the AD transducer (5) consists of a capacitor (9) which is connected to the AD transducer, and which, with an analogue switch (11) before it, is connected to the signal conditioning unit (4) via a resistor (10).

3. Circuit configuration according to claim 2, characterized by the fact, that the capacitor (9) for discharging and recharging can be controlled by the signal conditioning unit (4), which controls the charging and/or discharging processes of the capacitor (9) via the resistor (10) and the analogue switch (11) until the appropriate setpoint value for the zero point or for the sensitivity is reached.

4. Circuit configuration according to claim 1, characterized by the fact, that the sensitivity voltage controlled by the signal conditioning unit (4) is set in such a way that the measuring range plus an adjustable overshoot can be shown on the display (6).

5. Circuit configuration according to claim 1, characterized by the fact, that the signal conditioning unit (4) is a microcontroller.

6. Circuit configuration according to claim 1 for adjusting and matching an analogue signal which is variable in value and polarity to the AD transducer, characterized by the fact, that
a) control elements (7, 8) are provided for matching the analogue signal, each of which consists of a capacitor (9) which is connected to the AD transducer (5) and which, with an analogue switch (11) before it, is connected to the signal conditioning unit (4) via a resistor (10),
b) the signal conditioning unit (4) enables the voltage of the capacitor (9) to be measured via the AD transducer (5), and
c) the capacitor (9) for discharging and recharging can be controlled by the signal conditioning unit (4), the latter controlling the charging and/or discharging processes of the capacitor (9) via the resistor (10) and the analogue switch (11) until the value of the voltage measured matches the setpoint value of the analogue signal.
